# EUROPEAN PATENT SPECIFICATION

(11) **EP 1 322 775 B1**
(45) Date of publication and mention of the grant of the patent: **11.05.2011**
(21) Application number: 01972318.8
(22) Date of filing: 05.10.2001
(51) Int. Cl.: C12N 15/90, C12N 15/60, C12N 15/53, C12P 7/06, C12P 7/56, C12N 9/04, C12N 9/88, C12N 1/21

(54) **ETHANOL PRODUCTION**
ETHANOLHERSTELLUNG
PRODUCTION D'ETHANOL

(30) Priority: 06.10.2000 GB 0024554; 13.11.2000 US 247017 P
(43) Date of publication of application: 02.07.2003
(73) Proprietor: Elsworth Biotechnology Limited, Guilford, Surrey GU1 1BA (GB)
(72) Inventor: JAVED, Muhammad, Dagenham Essex RM8 1YB (GB); CUSDIN, Fiona, Horley Surrey RH6 8DZ (GB); MILNER, Paul, Ickenham Uxbridge UB10 8PN (GB); GREEN, Edward, Guildford Surrey GU4 8HB (GB)
(74) Representative: Wallis, Naomi Rachel
(86) International application number: PCT/GB2001/004434
(87) International publication number: WO 2002/029030

(56) References cited:
- WO-A-01/49865
- WO-A-88/09379
- US-A- 5 916 787
- DATABASE INTERNET [Online] March 2000 (2000-03) US DEPARTMENT OF ENERGY-OFFICE OF FUELS DEVELOPMENT: "http://bioenergy.ornl.gov/99summaries/fer mentation.htm" XP002192984
- DATABASE EMBL [Online] Accession No E02669, 8 October 1997 (1997-10-08) MURAKAMI S. ET AL: "DNA encoding Bacillus sp. L-lactic acid dehydrogenase" XP002168106
- DATABASE EMBL [Online] Accession No Z27089, 1 January 1994 (1994-01-01) CANNIO R. ET AL: "B. stearothermophilus (NCIMB 12403) adh-ht gene for alcohol dehydrogenase" XP002192985
- DATABASE EMBL [Online] Accession No Z29080, 9 June 1994 (1994-06-09) GAT O. ET AL: "B. stearothermophilus gene for xylanase T-6" XP002192986
- CHOU Y C ET AL: "H-215. INACTIVATION OF THE D-LACTATE DEHYDROGENASE GENE IN ZYMOMONAS MOBILIS THROUGH HOMOLOGOUS RECOMBINATION" ABSTRACTS OF THE GENERAL MEETING OF THE AMERICAN SOCIETY FOR MICROBIOLOGY, THE SOCIETY, WASHINGTON, DC, US, vol. 99, no. 370, 3 June 1999 (1999-06-03), page 370 XP001037633 ISSN: 1060-2011
- BOYLE-VAVRA SUSAN ET AL: "Cloning of the Staphylococcus aureus ddh gene encoding NAD+-dependent D-lactate dehydrogenase and insertional inactivation in a glycopeptide-resistant isolate." JOURNAL OF BACTERIOLOGY, vol. 179, no. 21, November 1997 (1997-11), pages 6756-6763, XP002192983 ISSN: 0021-9193
- INGRAM L O ET AL: "METABOLIC ENGINEERING OF BACTERIA FOR ETHANOL PRODUCTION" BIOTECHNOLOGY AND BIOENGINEERING, INTERSCIENCE PUBLISHERS, LONDON, GB, vol. 58, no. 2/3, 20 April 1998 (1998-04-20), pages 204-214, XP000999111 ISSN: 0006-3592

## Description

This invention relates to the production of ethanol as a product of bacterial fermentation. In particular this invention relates to a novel method of gene inactivation and gene expression based upon homologous recombination.

Many bacteria have the natural ability to metabolise simple sugars into a mixture of acidic and neutral fermentation products via the process of glycolysis. Glycolysis is the series of enzymatic steps whereby the six carbon glucose molecule is broken down, via multiple intermediates, into two molecules of the three carbon compound pyruvate. The glycolytic pathways of many bacteria produce pyruvate as a common intermediate. Subsequent metabolism of pyruvate results in a net production of NADH and ATP as well as waste products commonly known as fermentation products. Under aerobic conditions, approximately 95% of the pyruvate produced from glycolysis is consumed in a number of short metabolic pathways which act to regenerate NAD⁺ via oxidative metabolism, where NADH is typically oxidised by donating hydrogen equivalents via a series of steps to oxygen, thereby forming water, an obligate requirement for continued glycolysis and ATP production.

Under anaerobic conditions, most ATP is generated via glycolysis. Additional ATP can also be regenerated during the production of organic acids such as acetate. NAD⁺ is regenerated from NADH during the reduction of organic substrates such as pyruvate or acetyl CoA. Therefore, the fermentation products of glycolysis and pyruvate metabolism include organic acids, such as lactate, formate and acetate as well as neutral products such as ethanol. 1.

The majority of facultatively anaerobic bacteria do not produce high yields of ethanol either under aerobic or anaerobic conditions. Most facultative anaerobes metabolise pyruvate aerobically via pyruvate dehydrogenase (PDH) and the tricarboxylic acid cycle (TCA). Under anaerobic conditions, the main energy pathway for the metabolism of pyruvate is via the pyruvate-formate-lyase (PFL) pathway to give formate and acetyl-CoA. Acetyl-CoA is then converted to acetate, via phosphotransacetylase (PTA) and acetate kinase (AK) with the co-production of ATP, or reduced to ethanol via acetaldehyde dehydrogenase (AcDH) and alcohol dehydrogenase (ADH). In order to maintain a balance of reducing equivalents, excess NADH produced from glycolysis is re-oxidised to NAD⁺ by lactate dehydrogenase (LDH) during the reduction of pyruvate to lactate. NADH can also be re-oxidised by AcDH and ADH during the reduction of acetyl-CoA to ethanol but this is a minor reaction in cells with a functional LDH. Theoretical yields of ethanol are therefore not achieved since most acetyl CoA is converted to acetate to regenerate ATP and excess NADH produced during glycolysis is oxidised by LDH.

Ethanologenic microorganisms, such as *Zymomonas mobilis* and yeast, are capable of a second type of anaerobic fermentation commonly referred to as alcoholic fermentation in which pyruvate is metabolised to acetaldehyde and CO₂ by pyruvate decarboxylase (PDC). Acetaldehyde is then reduced to ethanol by ADH regenerating NADH⁺ Alcoholic fermentation results in the metabolism of 1 molecule of glucose to two molecules of ethanol and two molecules of CO₂. DNA which encodes both of these enzymes in *Z. mobilis* has been isolated, cloned and expressed recombinantly in hosts capable of producing high yields of ethanol via the synthetic route described above. For example; US 5,000,000 and Ingram et al (1997) Biotechnology and Bioengineering 58, Nos. 2 and 3 have shown that the genes encoding both PDC *(pdc)* and ADH *(adh)* from *Z. mobilis* can be incorporated into a "pet" operon which can be used to transform *Escherichia coli* strains resulting in the production of recombinant *E. coli* capable of co-expressing the *Z. mobilis pdc* and *adh.* This results in the production of a synthetic pathway re-directing *E. coli* central metabolism from pyruvate to ethanol during growth under both aerobic and anaerobic conditions. Similarly, US 5,554520 discloses that *pdc* and *adh* from *Z. mobilis* can both be integrated via the use of a pet operon to produce Gram negative recombinant hosts, including *Erwina, Klebsiella* and *Xanthomonas* species, each of which expresses the heterologous genes of *Z. mobilis* resulting in high yield production of ethanol via a synthetic pathway from pyruvate to ethanol.

US 5482846 discloses the simultaneous transformation of mesophilic Gram positive *Bacillus sp* with heterologous genes which encode both the PDC and ADH enzymes so that the transformed bacteria produce ethanol as a primary fermentation product. There is no suggestion that bacteria transformed with the *pdc* gene alone will produce ethanol.

EP-A-0761815 describes a method of homologous recombination whereby a sporulation gene is inserted into *Bacillus thurengiensis.*

EP-A-0603416 describes a method of homologous recombination whereby an arbitary gene is inserted into *Lactobacillus delbrueckii.*

EP-A-0415297 describes a method of producing *Bacillus* strains expressing a mutant protease.

Biwas et al., (J. Bacteriol., 175. 3628-3635, 1993) describes a method of homologous recombination whereby *Lactococcus lactis* has a chromosomal gene replaced by a plasmid carried modified copy. The method uses a thermosensitive plasmid and cannot be used to transform a thermophilic bacterium.

A key improvement in the production of ethanol using biocatalysts can be achieved with thermophilic microorganisms that operate at high temperature. The conversion rate of carbohydrates into ethanol is much faster. For example, ethanol productivity in a thermophilic *Bacillus* is up to ten-fold faster than a conventional yeast fermentation process which operates at 30°C. Consequently, a smaller production plant is required for a given volumetric productivity, thereby reducing plant construction costs. At high temperature, there is a reduced risk of contamination in the fermenter from other microorganisms, resulting in less downtime, increased plant productivity and a lower energy requirement for feedstock sterilisation. Moreover, fermentation cooling is not required, reducing operating costs further. The heat of fermentation helps to evaporate ethanol, which reduces the likelihood of growth inhibition from high ethanol concentrations, a common problem with most bacterial fermentations. Ethanol evaporation in the fermenter head space also facilitates product recovery.

The inventors' strain originates from a wild-type isolate that is a natural composting organism and far more suited for the conversion of sugars found in agricultural feedstocks to ethanol than traditional mesophilic microorganisms. The base strain possesses all the genetic machinery for the conversion of hexose and pentose sugars, and cellobiose to ethanol; the inventors have simply blocked the LDH pathway to increase ethanol yields. This process is called self-cloning and does not involve expression of foreign DNA. Consequently, the resulting organism does not fall under the safety regulations imposed on the use of genetically modified organisms (GMOs).

In contrast, conventional biocatalysts are either good ethanol producers unable to utilise pentose sugars or poor ethanol producers that can utilise pentose sugars. These organisms have been genetically modified using complex genetic techniques so that they can convert both hexose and pentose sugars to ethanol. However, there are doubts about the stability of these recombinant organisms and concerns over safety since such organisms fall under the GMO safety regulations. Moreover, recombinant mesophiles have expensive nutrient requirements and are sensitive to high salt concentrations and feedstock inhibitors.

The metabolic reactions leading to lactic acid formation (LDH pathway) have been blocked by chemical mutagenesis and the resulting strain TN is lactate negative and produces ethanol in high yield. However, the mutant strain is unstable and spontaneously reverts to the lactacte-producing wild-type. Revertants grow faster than the mutant at low pH and in high sugar concentrations, and rapidly 'take-over' in continuous culture. During 'take-over', the main fermentation product changes from ethanol to lactate.

The inventors initiated a molecular biology program to tackle the stability problem and gain a better insight into the genetic systems involved in ethanol formation. The inventors first developed genetic techniques to specifically manipulate the organism and a sporulation deficient mutant amenable to genetic manipulation was then selected in continuous culture. The inventors then sequenced several key metabolic genes; lactate dehydrogenase (*ldh*), lactase permease (*lp*), alcohol dehydrogenase (*adh*) and a novel insertion sequence located within the *ldh* gene. DNA sequence analysis of the *ldh* gene from the chemically mutated strain revealed that the gene had been inactivated by the insertion of a naturally occurring insertion sequence element (IE) (also referred to as an IS element) in the coding region of the gene. Transposition into (and out of) the *ldh* gene and subsequent gene inactivation is itself unstable, resulting in reversion.

The inventors determined that the IE sequence within the *ldh* gene provides a large area for homologous recombination. It was therefore proposed that the stability of the *ldh* mutation could be improved by integration of plasmid DNA into the IE sequence already present within the *ldh* gene of strain TN.

The stability of the *ldh* gene mutation was improved by specific homologous recombination between a plasmid and the insertion sequence within the *ldh* gene. The resulting strain is a sporulation deficient, facultatively anaerobic, Gram-positive *Bacillus* which exhibits improved ethanol production characteristics in continuous culture. Results show that this new type of mutant is completely stable and has superior growth characteristics and ethanol productivity than the first mutants generated by chemical mutagenesis.

Strain improvement has been achieved through a novel method of gene integration based on homologous recombination. The site of integration and plasmid for recombination can also be used to integrate and overexpress native or heterologous genes.

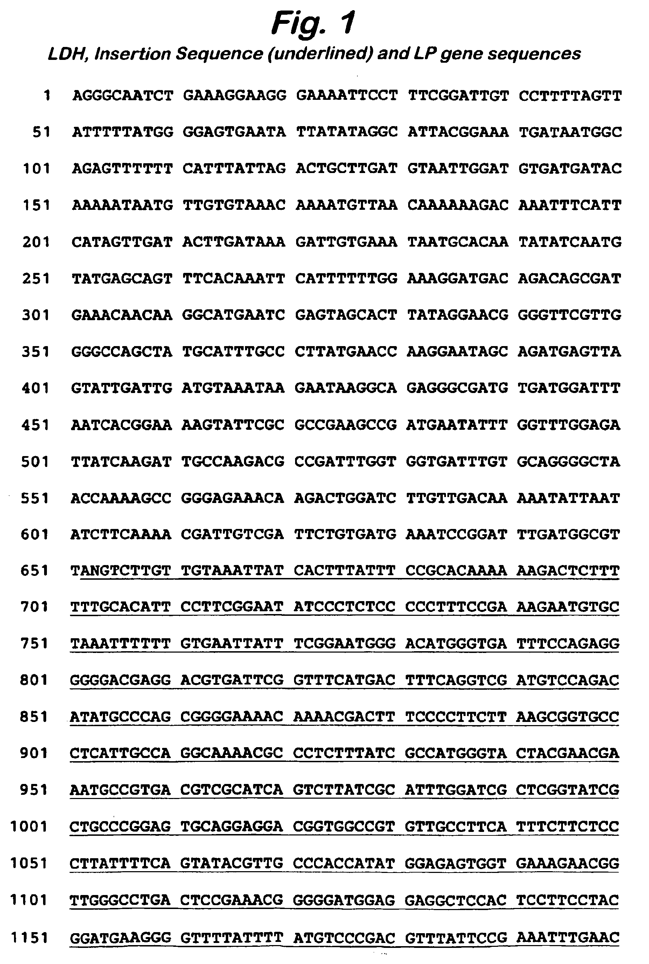

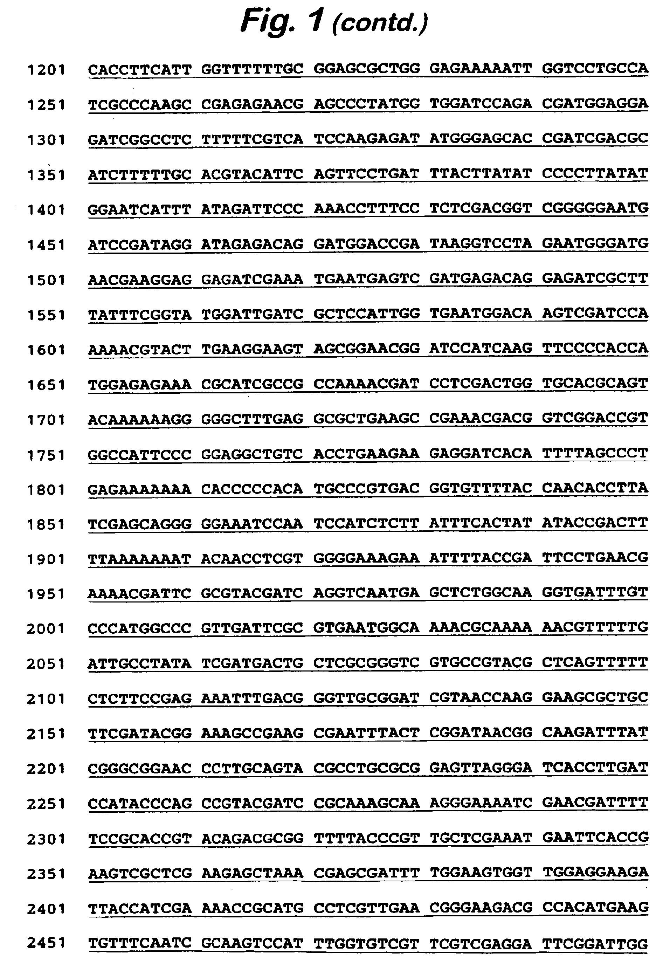

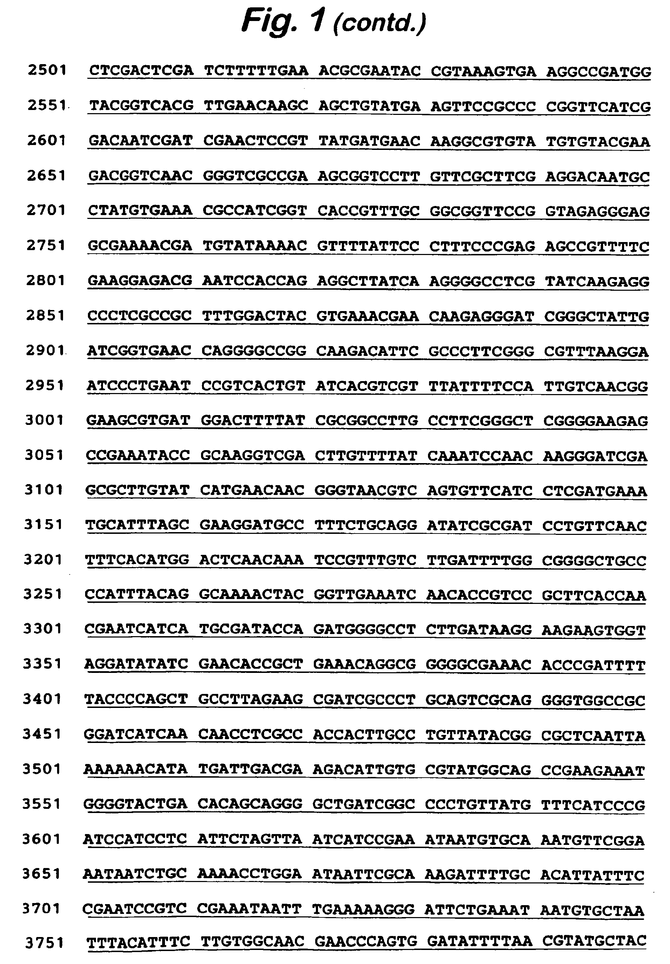

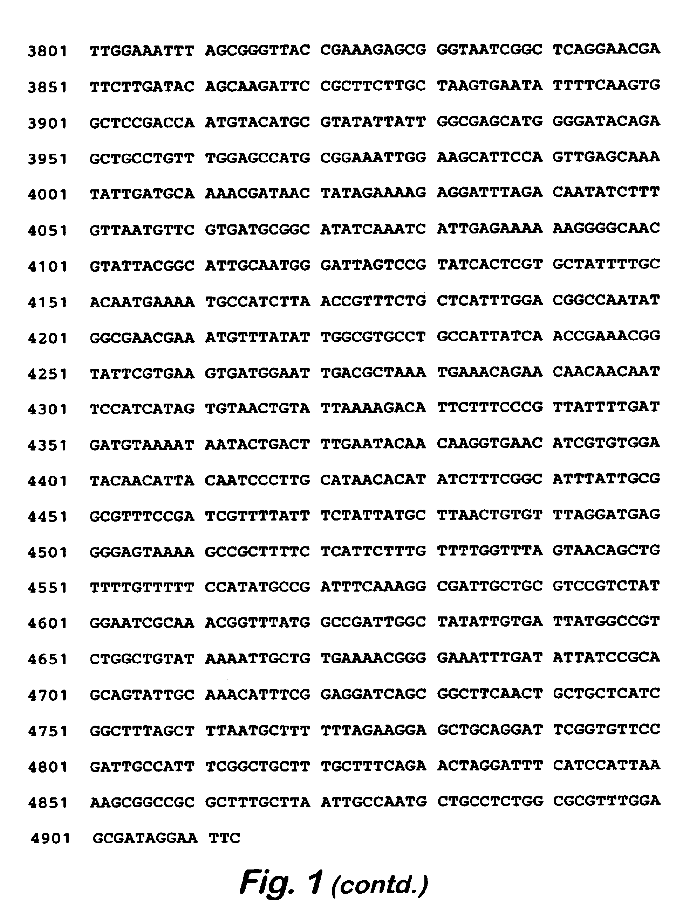

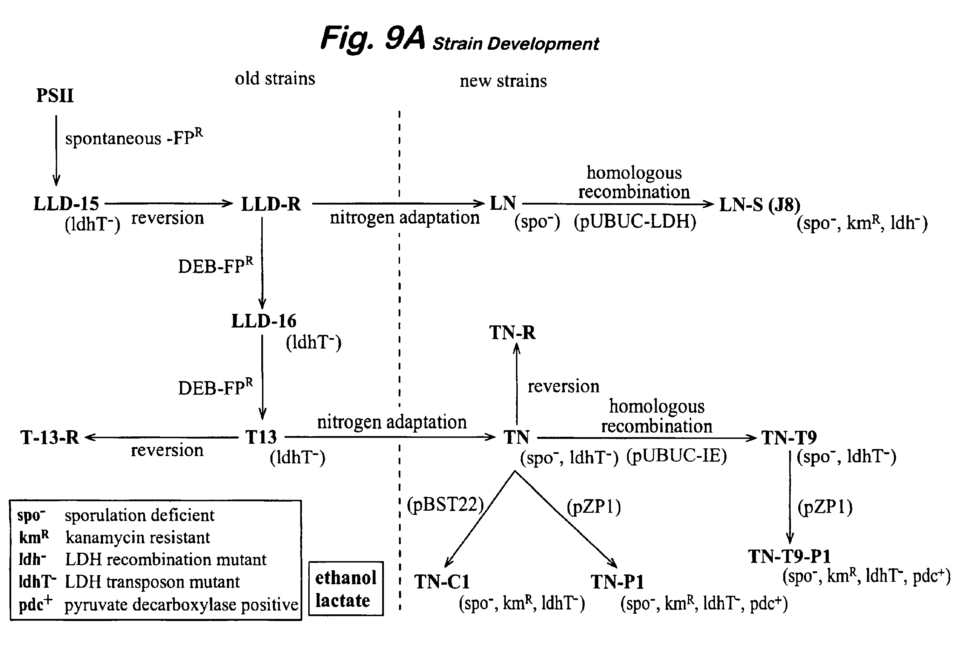

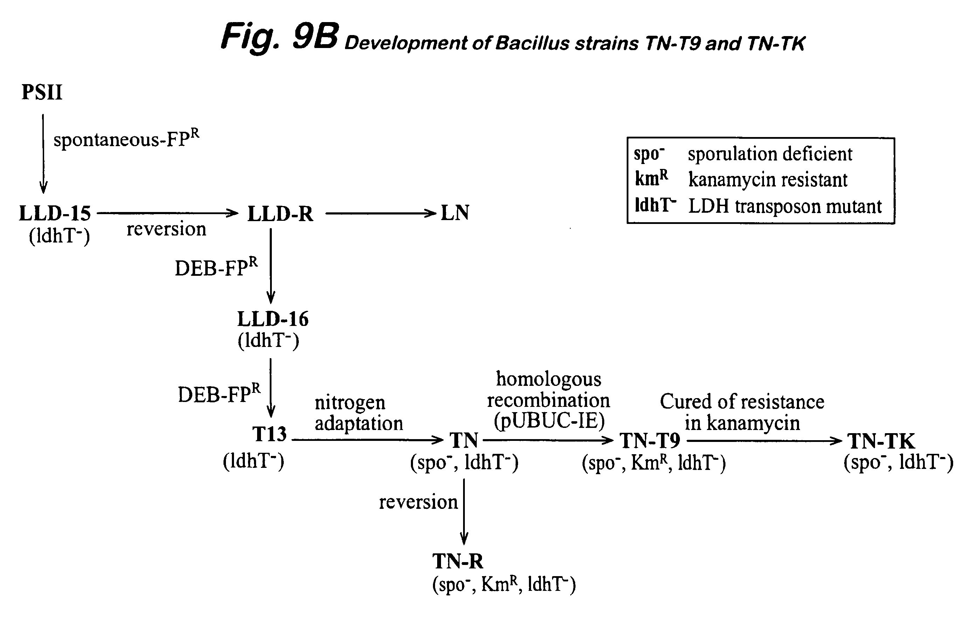

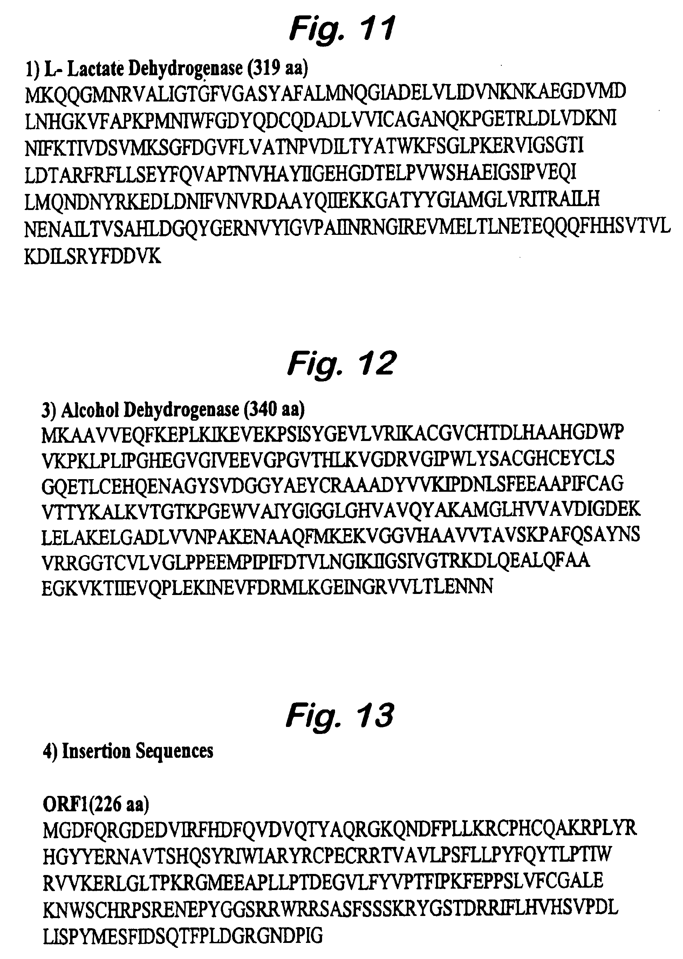

Southern hybridisation studies indicated that 3 copies of a transposable insertion sequence element (IE) are present on the chromosome of *Bacillus* strain LLD-R. The insertion sequence is 3.2kb long and comprises three DNA open reading frame sequences (ORF's) that are potentially translatable into proteins. ORF1 exhibits no homology to any protein in the National Center for Biotechnology Information (NCBI) database (www.ncbi.nlm.nih.gov/) whereas *ist*A and *ist*B display significant homology to a family of known transposase enzymes. *Bacillus* strain TN was developed from LLD-R following chemical mutagenesis (Figures 9A and 9B), and one copy of the insertion sequence was found within the structural *ldh* gene resulting in inactivation of the *ldh* gene and a lactate negative phenotype, the main metabolic product of fermentation thereby changing from lactate to ethanol. The DNA sequence of the *ldh* gene and the IE sequence (underlined) from *Bacillus* strain TN are shown in Fig. 1. The amino acid sequence of L-LDH is shown in Fig. 11.

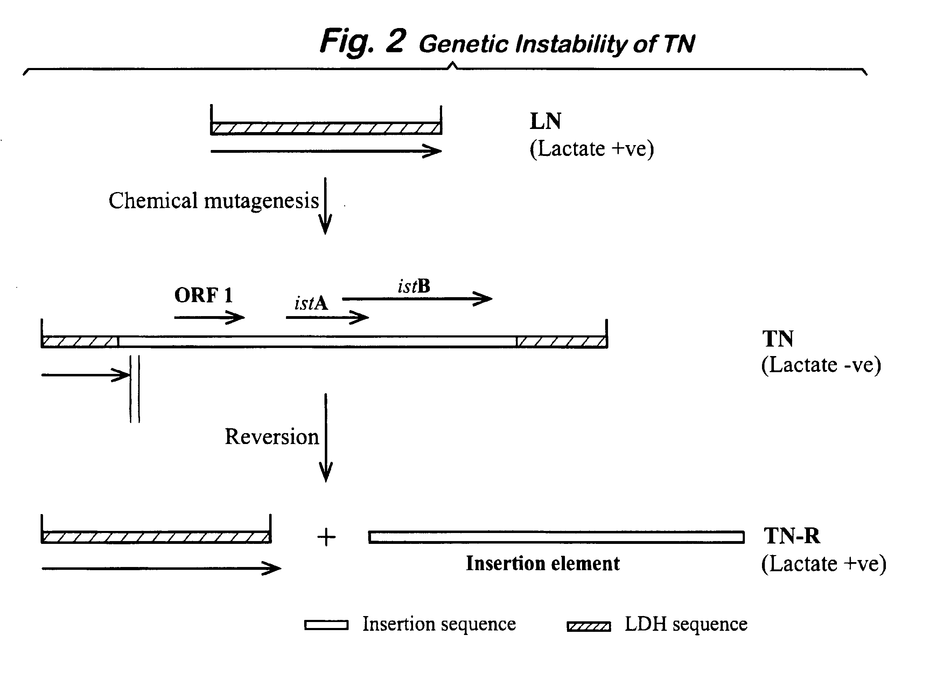

However, this insertion proved to be relatively unstable and the mutant strain TN spontaneously reverts back to strain TN-R with a functional *ldh* gene. The main metabolic product of fermentation changes from ethanol to lactate as shown in Fig. 2 which shows the genetic instability *of Bacillus* mutant strain TN.

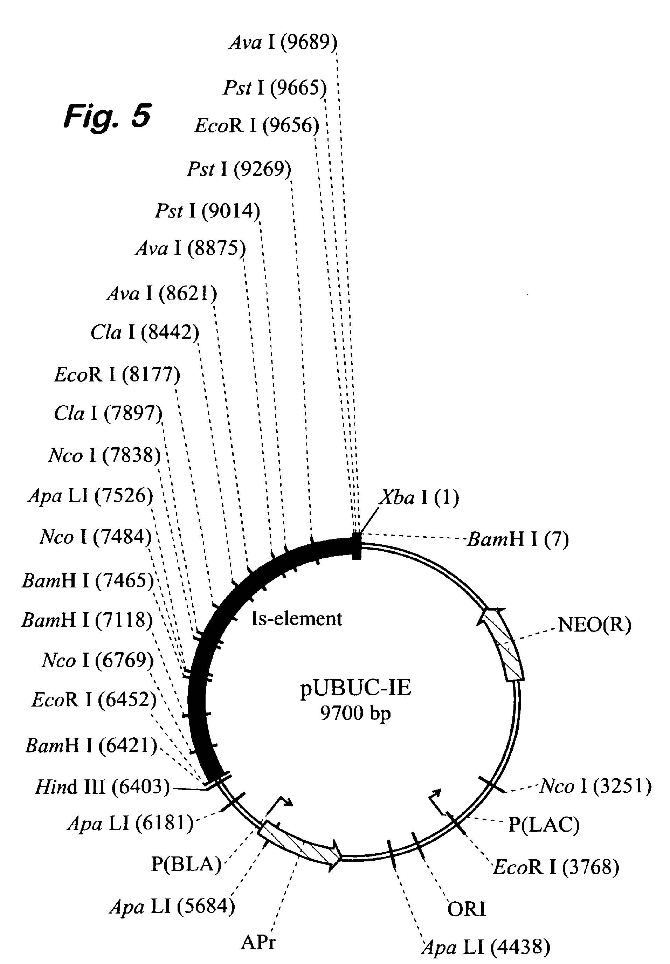

The IE sequence was amplified from TN chromosomal DNA by PCR. Primers were chosen from the *ldh* gene sequence that flanked the insertion sequence and a *Hind*III restriction site was introduced into the upstream primer and a *Xba*I restriction site was introduced into the downstream primer to create convenient restriction sites for subsequent cloning into plasmid pUBUC. A 3.2kb PCR fragment containing the insertion sequence was trimmed using *Hind*III and *Xba*I restriction endonucleases and subsequently cloned into plasmid pUBUC resulting in plasmid pUBUC-IE (Figure 5).

*In vivo* methylation of plasmid DNA to prevent its restriction after transformation of *Bacillus* sp. was achieved after transformation, propagation in and purification from *E*. *coli* TOP10 cells harbouring plasmid pMETH. Methylated pUBUC-IE was then used to transform *Bacillus* strain TN. Transformants were first isolated on TGP agar plates (kanamycin) at 52°C. Transformants were then screened using PCR amplification of the *ldh* gene. Failure to amplify a PCR product (greater than 10 kb using set PCR conditions) using LDH primers suggested that at least one copy of the plasmid had become integrated into the chromosome.

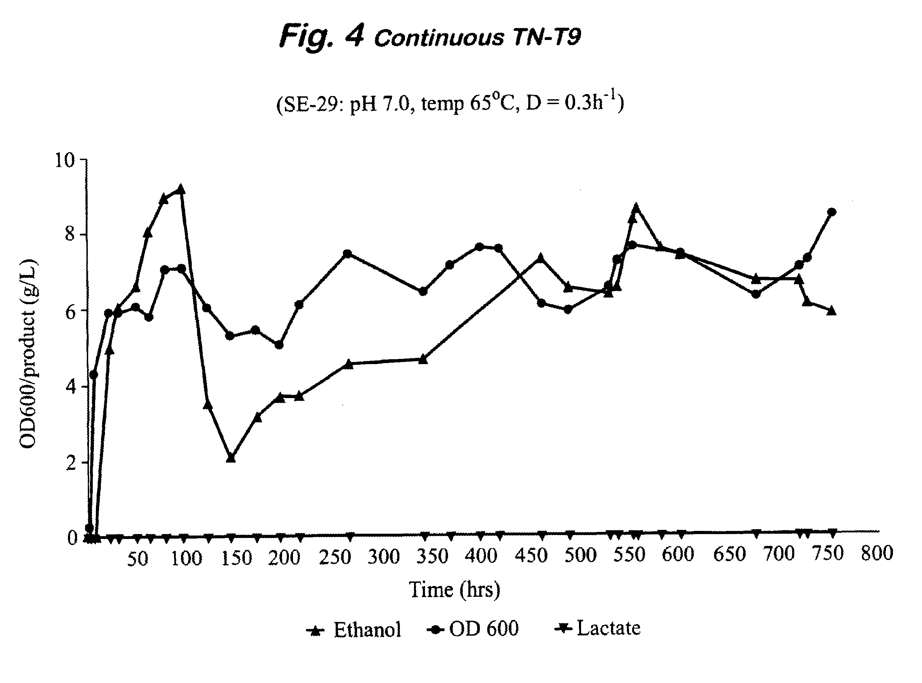

The new strain, TN-T9, was grown under pH controlled conditions in continuous culture without kanamycin selection to check for strain stability. Stability of strain TN-T9 was confirmed using sub-optimal fermentation conditions such that residual sugar was present within the fermentation medium; conditions which favour reversion. The fermentation ran continuously for 750 hours without any trace of lactate production despite the presence of residual sugar within the fermentation medium, pyruvate excretion and numerous deviations from the set conditions. Ethanol was produced in relatively large amounts throughout the fermentation Fig. 4, indicating that the *ldh* gene mutation in strain TN-T9 is stable in continuous culture under the experimental conditions provided.

The inventors have also optimised the fermentation conditions for cell growth and ethanol production for *Bacillus* strain TN-T9.

In summary the inventors have developed a dual system for improving the stability of the *ldh* mutant whilst expressing *pdc* and *adh* genes optionally using a *pdc*/*adh* operon. The inventors have also isolated and sequenced a novel *ldh* gene and insertion sequence element, as well as novel lactate permease and alcohol dehydrogenase genes. Furthermore, the inventors have developed a technique for the integration of plasmid DNA into the chromosome and selection of recombinant *Bacillus* sp and have developed a set of optimised conditions for the production of ethanol by bacterial fermentation.

Accordingly, a first aspect of the present disclosure relates to a recombinant thermophilic, Gram-positive bacterium which has been transformed using a method of homologous recombination for stabilising a gene mutation and for inserting an expressible gene..

Also provided is a recombinant thermophilic, Gram-positive bacterium in which the stability of the *ldh* mutation has been enhanced by homologous recombination between a plasmid and the chromosomal DNA of the bacterium resulting in a strain for the production of ethanol as a product of bacterial fermentation.

Preferably, the Gram-positive bacterium is a strain of *B. thermoglucosidasius.*

Preferably, the Gram-positive bacterium has been transformed with a plasmid harbouring an IE sequence as set forth in Fig. 1, or a functional portion or variant thereof. Advantageously, the IE sequence of Fig. 1, or functional variant or portion thereof, is stably incorporated into the chromosome of the recombinant bacterium by homologous recombination.

Preferably, integration of the IE sequence into the chromosome of the recombinant bacterium will result in the inactivation of the native *ldh* gene.

Preferably, the Gram-positive bacterium is *Bacillus* strain TN-T9 (NCIMB Accession no. NCIMB 41075 deposited on 8th September 2000 in accordance with the terms of the Budapest Treaty).

Alternatively, it is preferred that the Gram-positive bacterium is *Bacillus* strain TN-TK (NCIMB Accession no. NCIMB 41115 deposited on 27th September 2001 in accordance with the terms of the Budapest Treaty).

The present disclosure also relates to a Gram-positive bacterium obtained by selecting mutants of TN-T9 which are kanamycin sensitive. A suitable method for obtaining such strains is described in the appended examples.

Preferably, the Gram-positive bacterium is sporulation deficient.

According to a second aspect of the present disclosure there is provided a Gram-positive bacterium wherein a native *ldh* gene has been inactivated by homologous recombination and one or more expressible genes have been inserted into the chromosomal DNA of the bacterium. Furthermore, gene expression may be increased by increased gene copy number following multiple insertions of the plasmid into the insertion sequence either as a result of one round or repeated rounds of integration.

The one or more expressible genes may be inserted into one or more IE sequences present in the chromosomal DNA of the bacterium. For example, there are 3 IE sequences on the chromosome of strains TN-T9 and TN-TK.

The gene to be expressed may be native to *Bacillus* such as alcohol dehydrogenase or foreign (i.e. heterlogous such as pyruvate decarboxylase from Z. *mobilis* and α-amylase from *B. stearothermophilus.* The genes may also be arranged in an operon under the same transcriptional control. Gene expression may be regulated by manipulating the copy number of the gene and by using different transcriptional promoter sequences.

Preferably, the one or more genes are *pdc* and/or *adh.*

The amino acid sequence of *adh* is shown in Fig. 12.

According to a third aspect of the disclosure there is provided a method of inactivating a native *ldh* gene and inserting one or more expressible genes into the chromosome of a bacterium by homologous recombination.

Preferably the bacterium is a thermophilic Gram-positive bacterium.

Preferably, the gene to be inactivated is a native *ldh* gene and the one or more expressible genes are a *pdc* gene and a *adh* gene.

Preferably, the *pdc* gene and the *adh* gene form part of a PDC operon operatively linked to the IE sequence of Fig. 1 on the same plasmid.

Preferably the *pdc* gene is heterologous to the cell.

Preferably, both the IE sequence of Fig. 1 and the PDC operon, or portions thereof, are stably integrated into the chromosome of the bacterium.

Advantageously, the method of gene inactivation and expression comprises the use of a shuttle vector, as set forth in Fig. 5, which is able to replicate in *E. coli* and *Bacillus* strains at temperatures up to 54°C.

According to a fourth aspect of the present disclosure there is provided a shuttle vector which is able to replicate in both *E. coli* and *Bacillus* sp at temperatures up to 54°C, which confers resistance to ampicillin and kanamycin and which harbours the IE sequence, or a portion thereof as set forth in Fig. 1, from *Bacillus* strain TN.

Preferably, the shuttle vector is pUBUC-IE as set forth in Fig. 5.

Preferably, the shuttle vector will contain a PDC operon comprising a *pdc* gene and a *adh* gene under the control of the *ldh* promoter and operably linked to the IE sequence of Fig. 1.

According to a fifth aspect of the present disclosure there is provided a method of selecting for recombinant *Bacillus* sp at high temperature wherein plasmid DNA has been stably integrated into the *ldh* gene of the recombinant bacterium by homologous recombination, comprising use of PCR to select for recombinants that do not contain the native *ldh* gene and IE sequence.

Preferably, successful integration of the insertion sequence into the *ldh* gene will be indicated by failure to amplify a PCR product from the *ldh* gene of the recombinant bacterium.

The present invention also provides one or more polypeptides encoded by the sequence shown in Fig. 1 from nucleotide 652 to nucleotide 3800, or a functional variant or portion thereof, wherein the one or more polypeptides have the biological activity of a transposase.

The one or more polypeptides may have the biological activity of a transposase taken alone or when combined with other polypeptides.

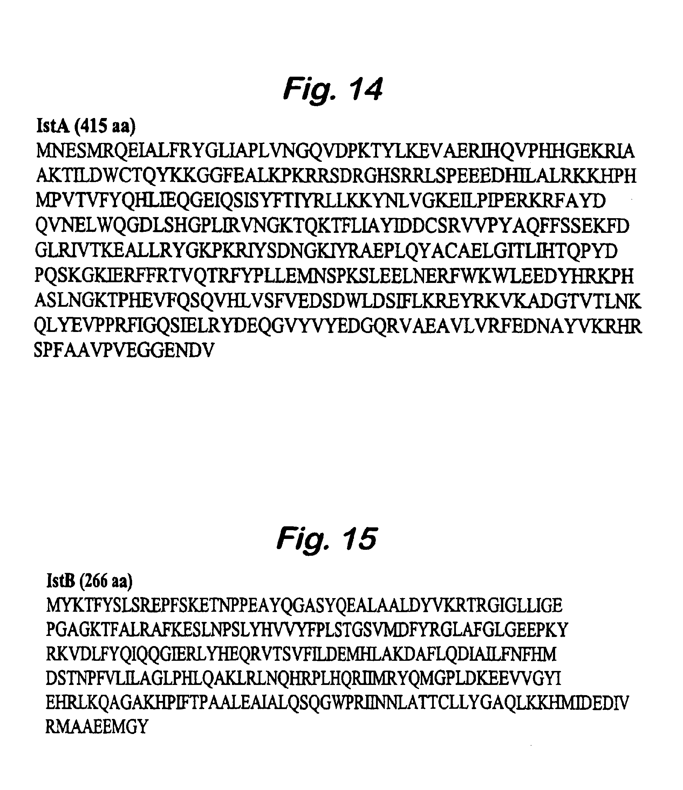

Preferably, the one or more polypeptides has the amino acid sequence shown in Fig. 13, Fig. 14 or Fig. 15 or a functional portion or variant thereof.

The functional portions or variants retain at least part of the transposase function of the polypeptide shown in Fig. 13, Fig. 14 or Fig. 15. Preferably the portions are at least 20, more preferably at least 50 amino acids in length. Furthermore, it is preferred that the variants have at least 80%, more preferably at least 90% and most preferably at least 95% sequence homology with the polypeptide shown in Fig. 13, Fig. 14 or Fig. 15. Homology is preferably measured using the BLAST program.

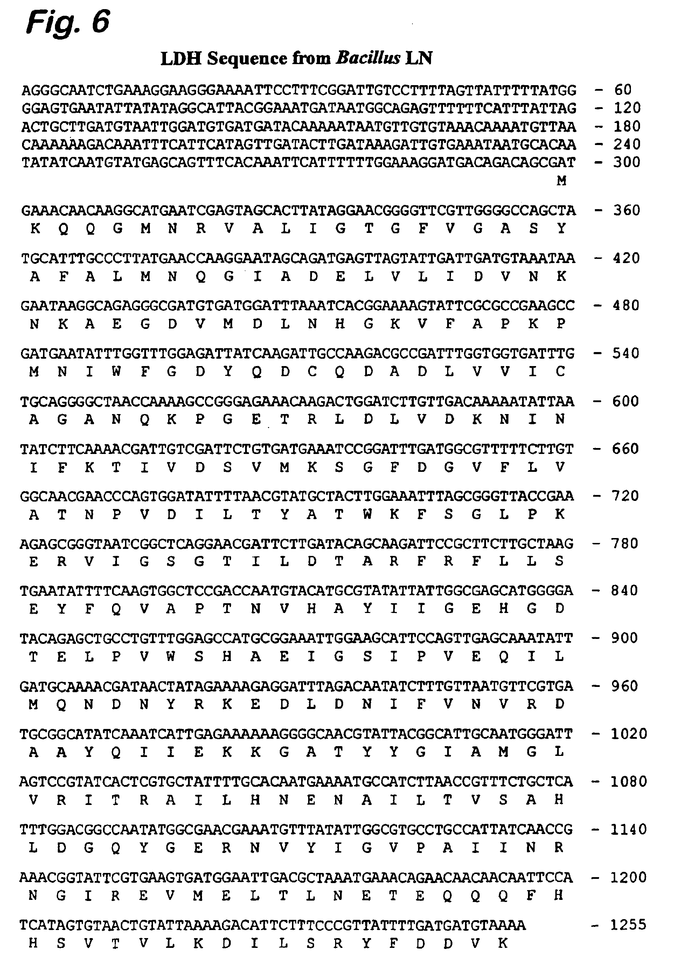

According to a sixth aspect of the disclosure there is provided a DNA sequence as set forth in Fig. 6, or a functional variant thereof, which codes for a polypeptide having the biological activity of the enzyme lactate dehydrogenase.

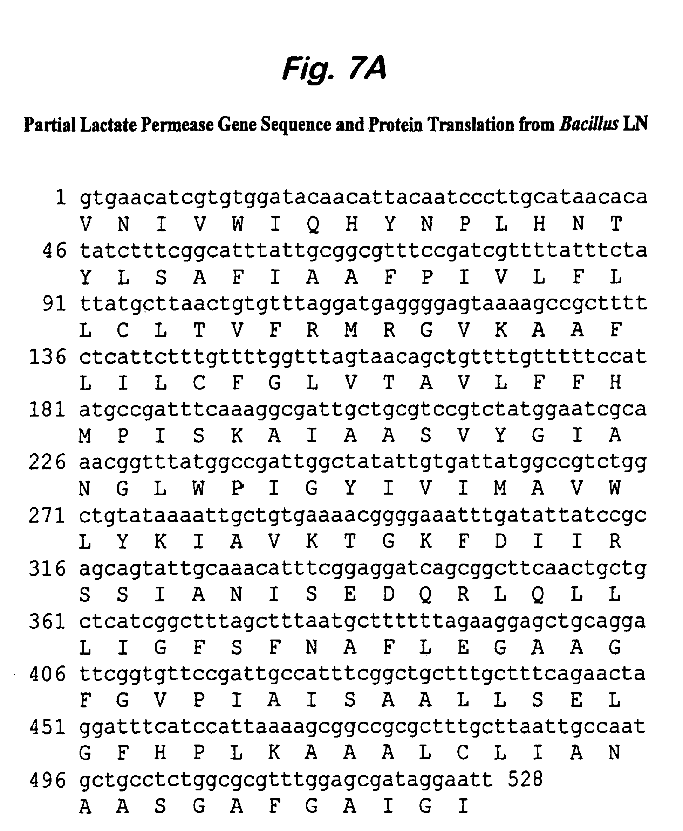

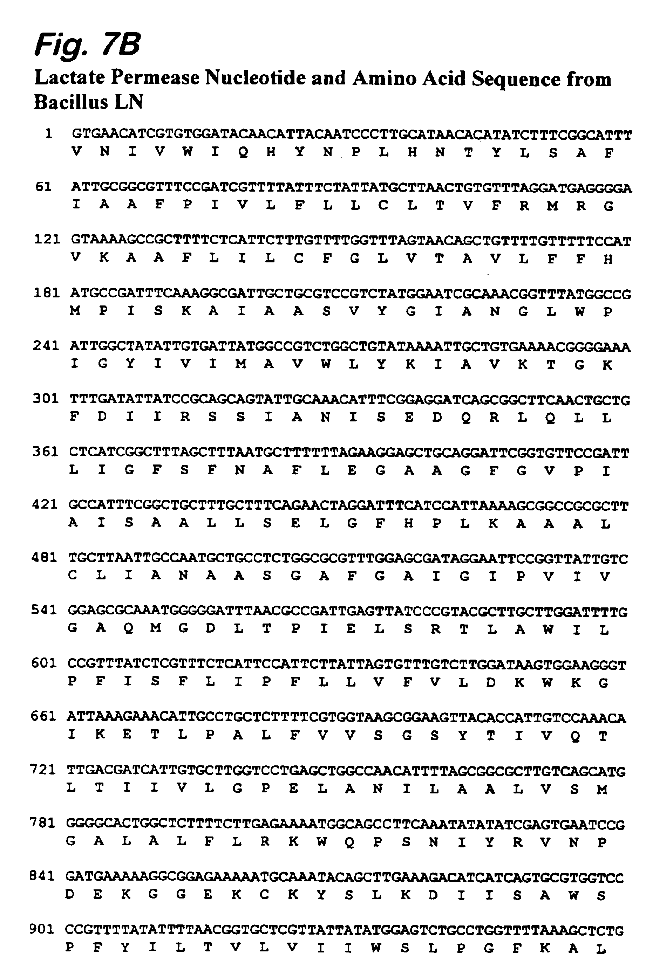

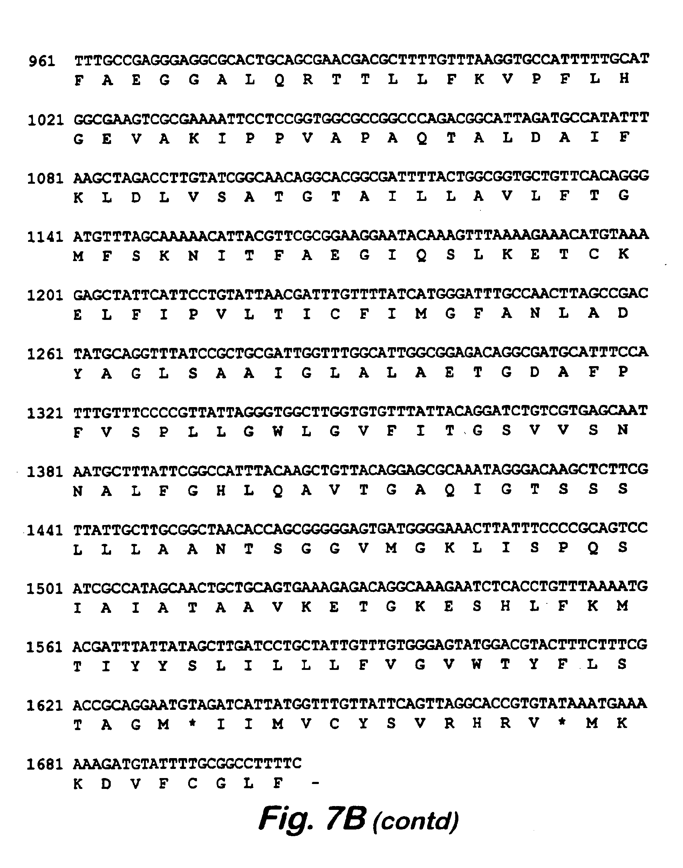

According to a seventh aspect of the present disclosure there is provided a DNA sequence as set forth in Fig. 7B, or a functional variant thereof, which codes for a polypeptide having the biological activity of the enzyme lactate permease.

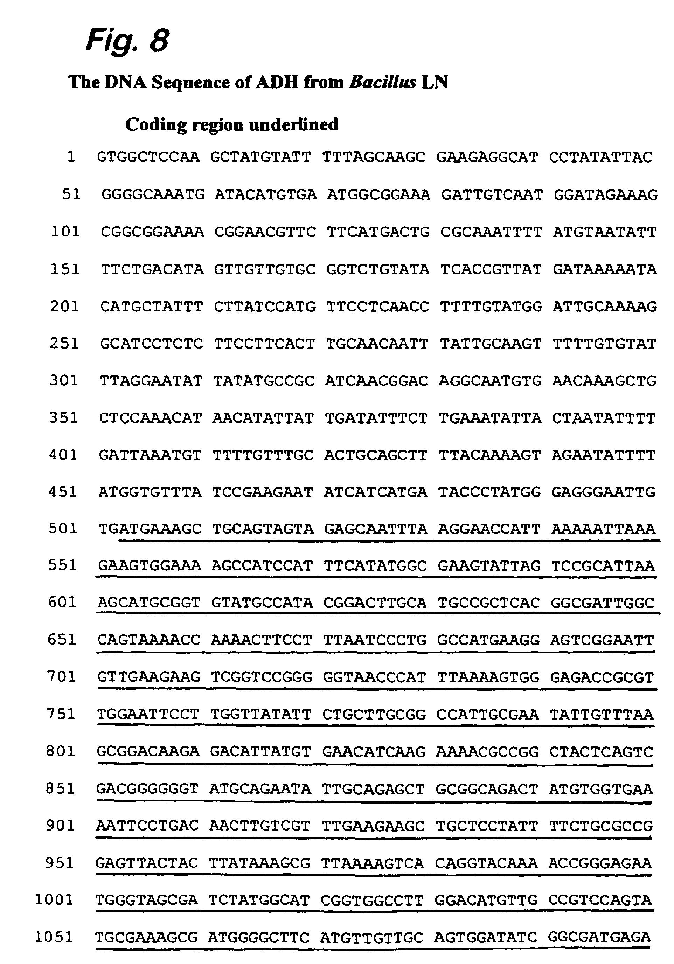

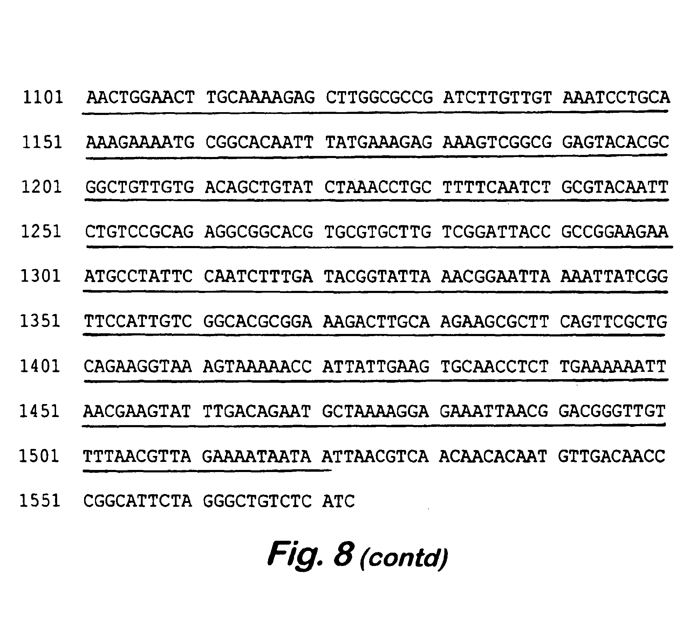

According to an eigth aspect of the present disclosure there is provided a DNA sequence as set forth in Fig. 8, or a functional variant thereof, which codes for a polypeptide having the biological activity of the enzyme alcohol dehydrogenase.

In this specification, functional variants include DNA sequences which as a result of sequence additions, deletions or substitutions, or which by virtue of the degeneracy of the genetic code, hybridise to and/or encode a polypeptide having a lactate dehydrogenase lactate permease or alcohol deydrongenase activity. Preferably, the variants have at least 80%, more preferably 90% and most preferably 95% sequence homology to the sequence shown in the Figures. Homology is preferably measured using the BLAST program.

A ninth aspect of the disclosure also provides a method for improving the stability of the *ldh* mutant comprising expressing genes using a *pdc*/*adh* operon.

A tenth aspect of the present disclosure relates to a technique for the integration and selection of recombinant *Bacillus* sp in accordance with the invention.

According to the final eleventh aspect of the present invention there is provided a process for the production of ethanol by bacterial fermentation of the Gram-positive bacterium of the present invention comprising optimised fermentation conditions of pH, temperature, redox values and specific dilution rates for cell growth and ethanol production. Preferably, the fermentation conditions will comprise a pH range of between 5.5-7.5 and a temperature range of 40-75°C with redox values being between - 360 - 400 mV and dilution rates between 0.3 and 0.8h⁻¹.

### Brief Description of the Drawings

The production of recombinant bacteria in accordance with the present invention will now be described, by way of example only, with reference to the drawings in which:
Fig. 1 shows the nucleotide sequence of a DNA sequence comprising an insertion element (IE), wherein the IE sequence is underlined;
Fig. 2 is a schematic representation of the genetic instability of strain TN;

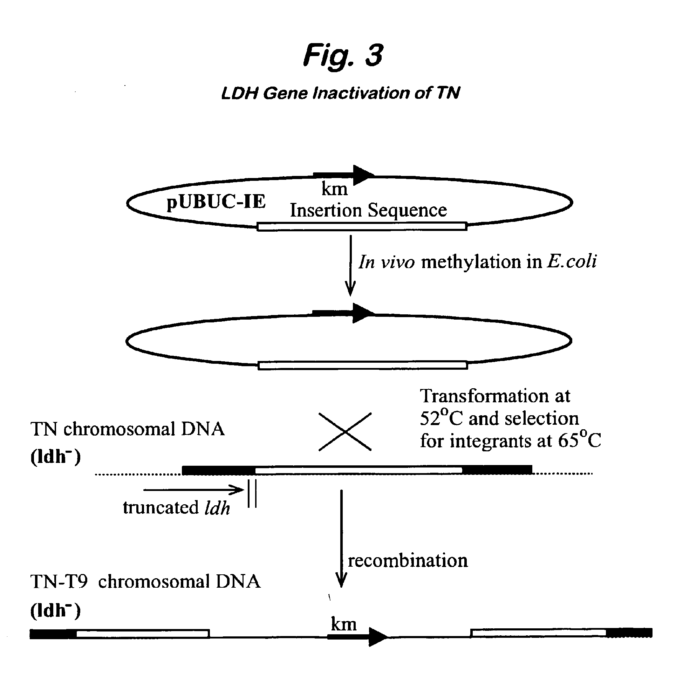

Fig. 3 is a schematic representation of the method for LDH gene inactivation by single-crossover recombination in *Bacillus* mutant strain TN;
Fig. 4 is a graphical representation showing the stability of *Bacillus* mutant strain TN-T9 in continuous culture for over 750 hours;
Fig. 5 is a schematic representation of shuttle vector pUBUC-IE;
Fig. 6 shows the DNA sequence of a novel lactate dehydrogenase gene and translated amino acid sequence from *Bacillus* strain LN;
Fig. 7A shows the partial DNA sequence of a novel lactate permease gene and the translated amino acid sequence from *Bacillus* strain LN;
Fig. 7B shows the full DNA sequence of a novel lactate permease gene and the translated amino acid sequence from *Bacillus* strain LN;Fig. 8 shows the DNA sequence of a novel alcohol dehydrogenase gene (underlined) from *Bacillus* strain LN;
Fig. 9 is a schematic representation showing (A) the development of *Bacillus* strain TN-T9 and (B) the development of *Bacillus* strains TN-T9 and TN-TK;

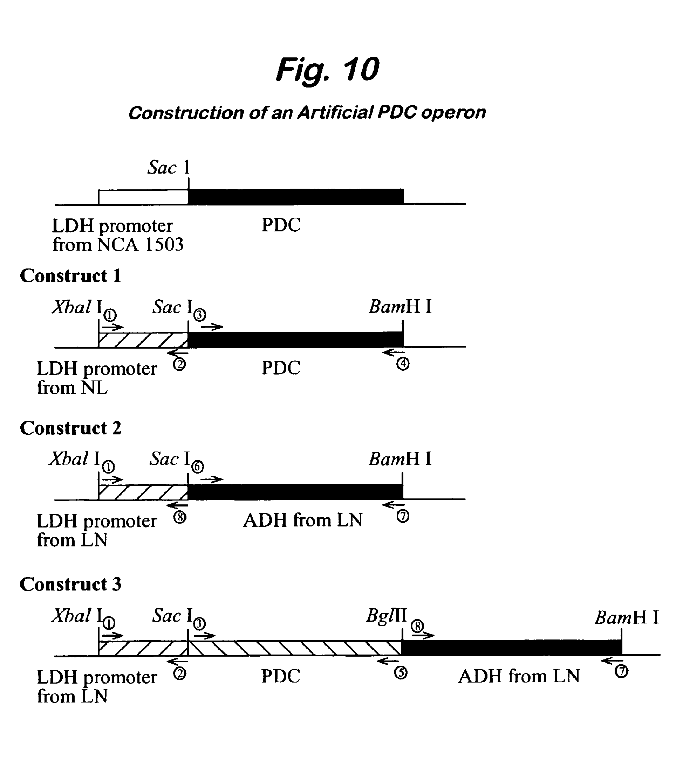

Fig. 10 shows the construction of an artificial PDC operon;
Fig. 11 shows the amino acid sequence of L-lactate dehydrogenase *(ldh)* from the TN strain;
Fig. 12 shows the amino acid sequence of alcohol dehydrogenase *(adh)* from the TN strain;
Fig. 13 shows the amino acid sequence of a transposase encoded by the IE sequence.
Fig. 14 shows the amino acid sequence of a transposase encoded by the IE sequence.
Fig. 15 shows the amino acid sequence of a transposase encoded by the IE sequence.

### Examples

### Materials and Methods

### Construction of plasmid pUBUC

A shuttle vector for the transfer of DNA between *E. coli* and the inventor's thermophilic *Bacillus* strains was developed by fusing plasmids pUC18 and pUB110. Plasmid pUB110 is a widely used vector that was isolated from *Staphyloccocus aureus* which confers resistance to kanamycin and which can replicate in *B. stearothermophilus* at temperatures up to 54°C Narumi et al., 1992 Biotechnology Techniques 6, No. 1. Plasmids pUB110 and pUC18 were linearised with *Eco*R1 and *Bam*H1, and then ligated together to form pUBUC (6.4kb). Plasmid pUBUC has a temperature sensitive replicon, and cannot replicate above 54°C making it an ideal host for gene integration, via homologous recombination at elevated temperatures.

### Construction of plasmid pMETH

A 1.1kb fragment containing the *met* gene was amplified from *Haemophilus aeygptius* chromosomal DNA by PCR. The gene was verified by DNA sequencing. The *met* gene was trimmed with *Bam*HI and *Xba*I, and then subcloned into the expression plasmid pCL1920, previously linearised with *Bam*H1 and *Xba*I*.* The resultant plasmid pMETH was transformed into *E. coli* TOP10. *E. coli* TOP10 cells harbouring pMETH were propagated and the culture was harvested for subsequent transformation and *in vivo* methylation using a method described by Tang et al (1994) Nuc. Acid Res. 22 (14). Competent cells were stored in convenient aliquots at -70°C prior to transformation.

### PCR Amplification

The IE sequence was amplified from TN chromosomal DNA by PCR using primers LDH7 and LDH8. The concentration of reactants and the PCR procedure used were those recommended in the Expand^{™} High Fidelity PCR System (Roche Diagnostics). PCR amplification from lyophilised cells was achieved after 30 cycles in a Genius thermocycler (Techne, Ltd., Cambridge). The sequence of the upstream primer, LDH7, was 5'-AAGCTT GAT GAA ATC CGG ATT TGA TGG-3' and the sequence of the downstream primer, LDH8 was 5'-TCTAGA GCT AAA TTT CCA AGT AGC-3'. These primers were chosen from the *ldh* gene sequence that flanked the insertion sequence. A *Hind*III restriction site was introduced into the upstream primer and a *Xba*I restriction site was introduced into the downstream primer to create convenient restriction sites for subsequent cloning (introduced sites are underlined).

### Construction of plasmid pUBUC - IE

The manipulation, transformation and isolation of plasmid DNA in *E. coli* was performed using standard procedures (Maniatis). A 3.2 kb PCR fragment containing the insertion sequence was trimmed with *Hind*III and *Xba*I and then cloned into plasmid pUBUC. The resulting shuttle plasmid, referred to as pUBUC-IE (Figure 5) can replicate in *E. coli* and *Bacillus* strains at temperatures up to 54°C, confers resistance to ampicillin and kanamycin, and harbours the IE sequence from *Bacillus* strain TN.

### Construction of PDC Operon

*Bacillus* strain TN converts the intracellular metabolite pyruvate to acetyl-CoA via the PFL or PDH pathway. Acetyl-CoA is then reduced to acetaldehyde and then to ethanol in reactions catalysed by AcDH and ADH, respectively. The introduction of a foreign PDC enzyme provides the cells with an alternative pathway for ethanol production that involves decarboxylation of pyruvate by PDC to form acetaldehyde which is then reduced to ethanol by the native ADH enzyme. Both PDC and ADH are involved in the conversion of pyruvate to ethanol.

We have shown that expression of *Z. mobilis pdc* from plasmid pZP-1 improves cell growth and stability of the mutant strain TN. However, we did not see any significant increase in ethanol formation. Therefore, we decided to increase *pdc* gene expression and co-express the native *adh* gene from *Bacillus* TN.

In plasmid pZP-1, the *pdc* gene was placed under the control of the *ldh* promoter sequence from *B. stearothermophilus* NCA1503. We decided to change the promoter with the *ldh* promoter from *Bacillus* LN (construct 1). We then placed the *adh* gene from *Bacillus* strain LN under the control of the *ldh* promoter (construct 2). Finally, both *pdc* (from Z. *mobilis* and *adh* (from *Bacillus* LN) were placed under the control of the *ldh* promoter sequence (construct 3). All the genes have been amplified by PCR from Z. *mobilis* (*pdc*) and *Bacillus* strain LN *(ldh* promoter and *pdc),* trimmed with the appropriate restriction enzymes, ligated together and cloned into an *E. coli* plasmid vector. The 3 constructs were cloned into the replicative shuttle vectors pUBUC, pFC1 or the integrative shuttle vector pUBUC-IE for chromosomal integration.

### Example 1

### Transformation of TN

Plasmid pUBUC-IE was methylated *in vivo* after transformation, propagation in and purification from *E. coli* TOP10 cells harbouring plasmid pMETH. Methylated pUBUC-IE was then used to transform *Bacillus* strain TN. *Bacillus* strain TN cells were grown at 65°C in 50ml of TGP medium until the absorbance at 600nm (A₆₀₀) reached 0.5-0.6. The culture was chilled on ice for 15-30 min. The cells were harvested by centrifugation and washed once in 10ml and twice in 5ml of cold TH buffer (272mM trehalose and 8mM HEPES; pH 7.5 with KOH). The cell pellet was re-suspended in 400µl of TH buffer and stored at 4°C prior to electroporation. Methylated plasmid DNA was used to transform strain TN by electroporation based on a method previously described by Narumi et al (1992) Biotechnology Techniques 6(1). The competent cells were dispensed into 90µl aliquots and mixed with 2µl of methylated plasmid DNA (250 ng/µl). The mixture was transferred to cold electroporation cuvettes (0.2cm electrode gap) and incubated on ice for 5 minutes. The suspensions were then subjected to a 2.5kV discharge from a 25µF capacitor and the pulse control was set at 201 ohms (time constant, τ = 5 ms) using a EquiBio EasyJect electroporator. The cells were immediately transferred to 5ml of pre-warmed TGP, incubated at 52°C for 1hr, and plated on TGP agar (10µg/ml kanamycin). The plates were incubated for 24-48 hours at 52°C.

### Selection of Recombinants

The following method was used to select for chromosomal integration of the temperature sensitive plasmid pUBUC-IE by homologous recombination.
1. Transformants were grown in 5ml of TGP (kanamycin) medium at 52°C for 24 hours.
2. 50ml of fresh TGP (kanamycin) medium was inoculated with 1ml from O/N culture and incubated in a shaking water bath at 52°C until a OD₆₀₀ was reached ~ 0.5.
3. 15ml of the above culture was centrifuged at 4100 rpm for 5 min at 5°C and the pellet was resuspended in 150µl of TGP (10:g/ml kanamycin) medium and spread on TGP (kanamycin) plates.
4. The plates were incubated at 68°C for 16 hours.
5. The isolated colonies were picked and analysed for plasmid integration into the insertion sequence site by PCR.

### Screening of TN integrants

TN integrants were isolated at 68°C. Failure to amplify a PCR product using LDH primers in TN integrants indicated that at least one copy of plasmid pUBUC-IE had become integrated intro the chromosome. As a result of integration the new strain TN-T9 was found to be more stable with regard to *ldh* reversion and "take over" than the parental strain TN.

### Stability of Strain TN-T9

The fermentation was run under sub-optimal conditions such that residual sugar was present in the medium; conditions which favour reversion. The fermentation ran continuously for over 750 hours without any trace of lactate production despite residual sugar, pyruvate excretion and numerous deviations from the set conditions. Ethanol was produced in relatively large amounts throughout the fermentation. Kanamycin was not used to select for integratnts throughout the entire fermentation. These results indicate that the *ldh* gene mutation in TN-T9 is stable in continuous culture under the experimental conditions (pH 7.0, 65°C with a 2% sugar feed).

### Ethanol Yields and Productivity

The fermentation conditions have been optimised for ethanol production from glucose, xylose and glucose/xylose based feedstocks.

| | |
|---|---|
| Culture type: | continuous |
| Temperature: | 65°C |
| pH: | 6.8 |
| Sugar concentration in feed: | 2-10% |
| Sparge gas: | air |
| Redox: | >-350mV (controlled through air flow rate) |
| Dilution rate: | 0.36-0.6 h⁻¹ |

Under these conditions the ethanol yields obtained were between 0.4-0.5 g/g sugar. Ethanol productivities, using a dilution rate of 0.5h⁻¹, were approximately 4 and 8 g ethanol/litre/hour on 2 and 4% sugar feeds, respectively.

### Example 2

### Selection of the kanamycin sensitive strain - TN-TK

*Bacillus* TN-TK is a kanamycin sensitive derivative of TN-T9. This strain is completely stable with regard to the *ldh* mutation and an ideal host for plasmid borne expression involving kanamycin as a selectable marker.

TN-T9 was first grown at 68°C for 24 hours in 5ml of TGP supplemented with kanamycin (10µg/ml). Approximately 100ml of culture was spread on two TGP (Km) agar plates and incubated overnight at 68°C. Several hundred colonies were obtained and 100 were transferred to fresh TGP (Km) plates using a sterile toothpick. After overnight growth at 68°C, the colonies were transferred (by replica plating) to fresh TGP plates and TGP (Km) plates and grown overnight at 68C.

Two kanamycin sensitive colonies were obtained on TGP but not on the corresponding TGP (Km) plate. The *ldh* gene regions from these colonies were amplified by PCR and found to be comparable in size to the disrupted *ldh* gene from TN-T9 (parental strain). PCR was used to demonstrate that the strains had lost the gene conferring resistance to kanamycin. One derivative referred to as TN-TK was chosen for further growth experiments. These experiments confirmed that the kanamycin sensitivity and *ldh* mutation were completely stable.

## Claims

1. A method of stabilising a *ldh* insertion mutation and inserting one or more expressible genes into the *ldh* gene comprising homologous recombination between an insertion sequence element (IE) within the native *ldh* gene and a plasmid comprising the insertion sequence element and the one or more expressible genes.

2. A method according to claim 1, wherein the insertion element sequence has the sequence set forth in figure 1.

3. A method according to claim 1 or claim 2, wherein the one or more expressible genes are a *pdc* gene and an *adh* gene.

4. The method according to claim 3 in which the *pdc* gene is from a *Zymomonas* sp.

5. The method according to claim 3 or 4 in which the heterologous *pdc* gene is from *Zymomonas mobilis.*

6. The method according to any one of claims 3 to 5 wherein the *adh* gene is from *Bacillus* strain LN.

7. The method according to any one of claims 3 to 6 wherein the insertion element sequence and a PDC operon, or functional portions thereof, are integrated in the chromosome of the recombinant bacterium.

8. The method according to any one of claim 3 to 7 comprising using a shuttle vector, as set forth in Fig. 5, which is able to replicate in *E*. *coli* and *Bacillus* strains.

9. A gram-positive bacterium having enhanced ethanol production characteristics, the bacterium comprising a stable inactivated *ldh* gene, the *ldh* gene comprising plasmid DNA comprising an insertion sequence element and one or more expressible genes within the insertion sequence element, wherein the bacterium is produced by the method of any of claims 1 to 8.

10. A Gram-positive bacterium according to claim 9, wherein the bacterium is a thermophile.

11. A Gram-positive bacterium according to claim 9, wherein the plasmid DNA comprises an expressible gene.

12. A Gram-positive bacterium according to claim 9, wherein the plasmid DNA comprises an insertion sequence element having the sequence set forth in figure 1.

13. A Gram-positive bacterium according to any of claims 9 to 12 wherein the bacterium is a Bacillus sp selected from B. stearothermophilus, B. caldovelox, B. caldotenax, B. thermoglucosidasius, B. coagulans, B. licheniformis, B. thermodenitrificans, and B. caldolyticus.

14. A Gram-positive bacterium according to any of claims 9 to 13 wherein the *Bacillus* sp is *B. thermoglucosidasius* strain TN-T9 deposited under NCIMB Accession No. NCIMB 41075.

15. A Gram-positive bacterium according to any of claims 9 to 13 wherein the *Bacillus* sp is *B. thermoglucosidasius* strain TN-TK deposited under NCIMB Accession No. NCIMB 41115.

16. A Gram-positive bacterium according to any of claims 9 to 15 wherein the bacterium has been transformed with a shuttle vector comprising the insertion element sequence, or a functional portion or variant thereof, as set forth in Fig. 1.

17. A Gram-positive bacterium according to any of claims 9 to 16 which is sporulation deficient.

18. A Gram-positive bacterium according to any of claims 9 to 17 which expresses a heterologous *pdc* gene.

19. A Gram-positive bacterium according to any of claims 9 to 18 wherein the *ldh* insertion mutation has been irreversibly stabilised.

20. A shuttle vector which harbours an insertion element sequence, or a functional fragment thereof, as set forth in Figure 1.

21. The shuttle vector according to claim 20 which harbours a *pdc* gene operatively linked to the IE sequence of Fig. 1.

22. The shuttle vector according to claim 21 wherein the *pdc* gene forms part of a PDC operon.

23. An isolated DNA molecule having a nucleotide sequence as set forth in Fig. 1 or a portion thereof, wherein the portion of the nucleotide sequence set forth in Fig. 1 codes for one or more polypeptides having the biological activity of a transposase and has been classified as an insertion sequence.

24. An isolated DNA molecule to claim 23, wherein the sequence is from thermophilic *Bacillus* strain TN.

25. One or more isolated polypeptides encoded by the sequence shown in Fig. 1 from residue 652 to residue 3800 or a portion thereof, wherein the one or more polypeptides have the biological activity of a transposase.

26. A process for the production of ethanol by bacterial fermentation of the Gram-positive bacterium according to any one of claims 9 to 19 comprising using optimised fermentation conditions wherein the pH of the fermentation medium is within the range of pH 5.5 - 7.5.

27. The process according to claim 26 wherein the pH of the fermentation medium is preferably between pH 6.0 - 7.0.

28. The process according to claim 26 or 27 wherein the pH of the fermentation medium is preferably between pH 6.4 - 6.9.

29. A process for the production of ethanol by bacterial fermentation of the Gram-positive bacterium according to any one of claims 9 to 19 comprising using optimised fermentation conditions wherein the temperature of the fermentation medium is within the range 40 - 75°C.

30. The process according to claim 29 wherein the temperature of the fermentation medium is preferably between 52 - 70°C.

31. The process according to claim 29 or 30 wherein the temperature of the fermentation medium is preferably between 60 - 68°C.

32. A process for the continuous production of ethanol by bacterial fermentation of the Gram-positive bacterium according to any one of claims 9 to 19 in which the feed dilution rates are between 0.3-0.8 h⁻¹.

33. A process according to claim 32 wherein the feed dilution rates are preferably between 0.4 - 0.6 h⁻¹.

34. A process according to any one of claims 26 to 33 in which the bacterium used is *Bacillus* strain TN.

## Patentansprüche

1. Verfahren zum Stabilisieren einer ldh-Insertionsmutation und Einfügen eines oder mehrerer exprimierbarer Gene in das ldh-Gen, umfassend eine homologe Rekombination zwischen einem Insertionssequenzelement (IE) innerhalb des nativen *ldh*-Gens und einem Plasmid, welches das Insertionssequenzelement und die ein oder mehreren exprimierbaren Gene umfasst.

2. Verfahren gemäß Anspruch 1, wobei die Insertionselementsequenz die in Figur 1 angegebene Sequenz aufweist.

3. Verfahren gemäß Anspruch 1 oder Anspruch 2, wobei die ein oder mehreren exprimierbaren Gene ein pdc-Gen und ein adh-Gen sind.

4. Verfahren gemäß Anspruch 3, wobei das pdc-Gen aus einer *Zymomonas* sp stammt.

5. Verfahren gemäß Anspruch 3 oder 4, wobei das heterologe pdc-Gen aus *Zymomonas mobilis* stammt.

6. Verfahren gemäß einem der Ansprüche 3 bis 5, wobei das adh-Gen aus Bacillus-Stamm LN stammt.

7. Verfahren gemäß einem der Ansprüche 3 bis 6, wobei die Insertionselementsequenz und ein PDC-Operon, oder funktionelle Abschnitte davon, in das Chromosom des rekombinanten Bakteriums integriert sind.

8. Verfahren gemäß einem der Ansprüche 3 bis 7, umfassend, einen Shuttle-Vektor, wie in Fig. 5 angegeben, zu verwenden, der zum Replizieren in *E.-coli*- und *Bacillus-*Stämmen fähig ist.

9. Grampositives Bakterium, das verbesserte Ethanolherstellungs-Eigenschaften aufweist, wobei das Bakterium ein stabiles inaktiviertes *ldh*-Gen umfasst, wobei das *ldh*-Gen Plasmid-DNA umfasst, die ein Insertionssequenzelement und ein oder mehrere exprimierbare Gene innerhalb des Insertionssequenzelementes umfasst, wobei das Bakterium durch das Verfahren gemäß einem der Ansprüche 1 bis 8 hergestellt ist.

10. Grampositives Bakterium gemäß Anspruch 9, wobei das Bakterium ein thermophiles Bakterium ist.

11. Grampositives Bakterium gemäß Anspruch 9, wobei die Plasmid-DNA ein exprimierbares Gen umfasst.

12. Grampositives Bakterium gemäß Anspruch 9, wobei die Plasmid-DNA ein Insertionssequenzelement umfasst, das die in Figur 1 angegebene Sequenz aufweist.

13. Grampositives Bakterium gemäß einem der Ansprüche 9 bis 12, wobei das Bakterium ein Bacillus sp ist, das aus B. stearothermophilus, B. caldovelox, B. caldotenax, B. thermoglucosidasius, B. coagulans, B. licheniformis, B. thermodenitrificans und B. caldolyticus ausgewählt ist.

14. Grampositives Bakterium gemäß einem der Ansprüche 9 bis 13, wobei das *Bacillus* sp *B. thermoglucosidasius-Stamm* TN-T9, hinterlegt unter NCIMB-Akzessionsnr. NCIMB 41075, ist.

15. Grampositives Bakterium gemäß einem der Ansprüche 9 bis 13, wobei das *Bacillus* sp *B. thermoglucosidasius-Stamm* TN-TK, hinterlegt unter NCIMB-Akzessionsnr. NCIMB 41115, ist.

16. Grampositives Bakterium gemäß einem der Ansprüche 9 bis 15, wobei das Bakterium mit einem Shuttle-Vektor transformiert worden ist, der die Insertionselementsequenz oder einen funktionellen Abschnitt oder eine Variante davon, wie in Fig. 1 angegeben, umfasst.

17. Grampositives Bakterium gemäß einem der Ansprüche 9 bis 16, das sporulationsdefizient ist.

18. Grampositives Bakterium gemäß einem der Ansprüche 9 bis 17, das ein heterologes *pdc*-Gen exprimiert.

19. Grampositives Bakterium gemäß einem der Ansprüche 9 bis 18, wobei die *ldh*-Insertionsmutation irreversibel stabilisiert worden ist.

20. Shuttle-Vektor, der eine Insertionselementsequenz oder ein funktionelles Fragment davon, wie in Figur 1 angegeben, beherbergt.

21. Shuttle-Vektor gemäß Anspruch 20, der ein *pdc*-Gen beherbergt, das operativ mit der IE-Sequenz aus Fig. 1 verbunden ist.

22. Shuttle-Vektor gemäß Anspruch 21, wobei das *pdc*-Gen einen Teil eines PDC-Operons bildet.

23. Isoliertes DNA-Molekül, das eine Nukleotidsequenz wie in Fig. 1 angegeben oder einen Abschnitt davon aufweist, wobei der Abschnitt der in Fig. 1 angegebenen Nukleotidsequenz für ein oder mehrere Polypeptide codiert, welche die biologische Aktivität einer Transposase aufweisen, und als eine Insertionssequenz klassifiziert worden ist.

24. Isoliertes DNA-Molekül gemäß Anspruch 23, wobei die Sequenz aus thermophilem *Bacillus*-Stamm TN stammt.

25. Ein oder mehrere isolierte Polypeptide, die durch die in Fig. 1 gezeigte Sequenz von Rest 652 bis Rest 3800 oder einen Abschnitt davon codiert sind, wobei die ein oder mehreren Polypeptide die biologische Aktivität einer Transposase aufweisen.

26. Prozess für die Herstellung von Ethanol durch bakterielle Fermentation des grampositiven Bakteriums gemäß einem der Ansprüche 9 bis 19, umfassend, optimierte Fermentationsbedingungen zu verwenden, wobei der pH-Wert des Fermentationsmediums im Bereich von pH 5,5 - 7,5 ist.

27. Prozess gemäß Anspruch 26, wobei der pH-Wert des Fermentationsmediums bevorzugt zwischen pH 6,0 - 7,0 ist.

28. Prozess gemäß Anspruch 26 oder 27, wobei der pH-Wert des Fermentationsmediums bevorzugt zwischen pH 6,4 - 6,9 ist.

29. Prozess für die Herstellung von Ethanol durch bakterielle Fermentation des grampositiven Bakteriums gemäß einem der Ansprüche 9 bis 19, umfassend, optimierte Fermentationsbedingungen zu verwenden, wobei die Temperatur des Fermentationsmediums im Bereich von 40 - 75°C ist.

30. Prozess gemäß Anspruch 29, wobei die Temperatur des Fermentationsmediums bevorzugt zwischen 52 - 70°C ist.

31. Prozess gemäß Anspruch 29 oder 30, wobei die Temperatur des Fermentationsmediums bevorzugt zwischen 60 - 68°C ist.

32. Prozess für die kontinuierliche Herstellung von Ethanol durch bakterielle Fermentation des grampositiven Bakteriums gemäß einem der Ansprüche 9 bis 19, wobei die Nährstoff-Verdünnungsraten zwischen 0,3 - 0,8 h⁻¹ sind.

33. Prozess gemäß Anspruch 32, wobei die Nährstoff-Verdünnungsraten bevorzugt zwischen 0,4 - 0,6 h⁻¹ sind.

34. Prozess gemäß einem der Ansprüche 26 bis 33, wobei das verwendete Bakterium *Bacillus*-Stamm TN ist.

## Revendications

1. Procédé pour stabiliser une mutation par insertion de *ldh* et insérer un ou plusieurs gènes aptes à l'expression dans le gène *ldh*, comprenant une recombinaison homologue entre un élément de séquence d'insertion (IE) à l'intérieur du gène *ldh* naturel et un plasmide comprenant l'élément de séquence d'insertion et le ou les gènes aptes à l'expression.

2. Procédé suivant la revendication 1, dans lequel la séquence d'éléments d'insertion a la séquence représentée sur la figure 1.

3. Procédé suivant la revendication 1 ou la revendication 2, dans lequel le ou les gènes aptes à l'expression consistent en un gène *pdc* et un gène *adh*.

4. Procédé suivant la revendication 3, dans lequel le gène *pdc* provient d'un *Zymomonas* sp.

5. Procédé suivant la revendication 3 ou 4, dans lequel le gène *pdc* hétérologue provient de *Zymomonas mobilis.*

6. Procédé suivant l'une quelconque des revendications 3 à 5, dans lequel le gène *adh* provient de la souche de *Bacillus* LN.

7. Procédé suivant l'une quelconque des revendications 3 à 6, dans lequel la séquence d'éléments d'insertion et un opéron PDC, ou les portions fonctionnelles de celui-ci, sont intégrés dans le chromosome de la bactérie recombinante.

8. Procédé suivant l'une quelconque des revendications 3 à 7, comprenant l'utilisation d'un vecteur alternatif, de la manière représentée sur la figure 5, qui est apte à la réplication dans *E*. *coli* et des souches de *Bacillus.*

9. Bactérie Gram-positive ayant des caractéristiques améliorées de production d'éthanol, la bactérie comprenant un gène *ldh* inactivé stable, le gène *ldh* comprenant un ADN de plasmide comprenant un élément de séquence d'insertion et un ou plusieurs gènes aptes à l'expression à l'intérieur de l'élément de séquence d'insertion, ladite bactérie étant produite par le procédé de l'une quelconque des revendications 1 à 8.

10. Bactérie Gram-positive suivant la revendication 9, ladite bactérie étant une bactérie thermophile.

11. Bactérie Gram-positive suivant la revendication 9, dans laquelle l'ADN de plasmide comprend un gène apte à l'expression.

12. Bactérie Gram-positive suivant la revendication 9, dans laquelle l'ADN de plasmide comprend un élément de séquence d'insertion ayant la séquence représentée sur la figure 1.

13. Bactérie Gram-positive suivant l'une quelconque des revendications 9 à 12, ladite bactérie étant un Bacillus sp choisi entre B. stearothermophilus, B. caldovelox, B. caldotenax, B. thermoglucosidasius, B. coagulans, B. licheniformis, B. thermodenitrificans et B. caldolyticus.

14. Bactérie Gram-positive suivant l'une quelconque des revendications 9 à 13, dans laquelle le *Bacillus* sp est la souche de *B. thermoglucosidasius* TN-T9 déposée sous le numéro de dépôt NCIMB NCIMB 41075.

15. Bactérie Gram-positive suivant l'une quelconque des revendications 9 à 13, dans laquelle le *Bacillus* sp est la souche de *B. thermoglucosidasius* TN-TK déposée sous le numéro de dépôt NCIMB NCIMB 41115.

16. Bactérie Gram-positive suivant l'une quelconque des revendications 9 à 15, ladite bactérie ayant été transformée avec un vecteur alternatif comprenant la séquence d'éléments d'insertion, ou une portion fonctionnelle ou un variant de celle-ci, de la manière représentée sur la figure 1.

17. Bactérie Gram-positive suivant l'une quelconque des revendications 9 à 16, qui est déficiente du point de vue de la sporulation.

18. Bactérie Gram-positive suivant l'une quelconque des revendications 9 à 17, qui exprime un gène *pdc* hétérologue.

19. Bactérie Gram-positive suivant l'une quelconque des revendications 9 à 18, dans laquelle la mutation par insertion de *ldh* a été stabilisée de manière irréversible.

20. Vecteur alternatif qui porte une séquence d'éléments d'insertion, ou un fragment fonctionnel de celle-ci, de la manière représentée sur la figure 1.

21. Vecteur alternatif suivant la revendication 20, qui porte un gène *pdc* lié de manière fonctionnelle à la séquence IE de la figure 1.

22. Vecteur alternatif suivant la revendication 21, dans lequel le gène *pdc* fait partie d'un opéron PDC.

23. Molécule d'ADN isolée ayant une séquence de nucléotides représentée sur la figure 1 ou une portion de celle-ci, dans laquelle la portion de la séquence de nucléotides représentée sur la figure 1 code pour un ou plusieurs polypeptides ayant l'activité biologique d'une transposase et a été classée en tant que séquence d'insertion.

24. Molécule d'ADN isolée suivant la revendication 23, dans laquelle la séquence provient de la souche TN de *Bacillus* thermophile.

25. Un ou plusieurs polypeptides isolés codés par la séquence représentée sur la figure 1, du résidu 652 au résidu 3800 ou une portion de celle-ci, ledit ou lesdits polypeptides ayant l'activité biologique d'une transposase.

26. Procédé pour la production d'éthanol par fermentation bactérienne de la bactérie Gram-positive suivant l'une quelconque des revendications 9 à 19, comprenant l'utilisation de conditions optimisées de fermentation dans lesquelles le pH du milieu de fermentation est compris dans la plage de pH 5,5 à 7,5.

27. Procédé suivant la revendication 26, dans lequel le pH du milieu de fermentation est compris de préférence entre pH 6,0 et 7,0.

28. Procédé suivant la revendication 26 ou 27, dans lequel le pH du milieu de fermentation est compris de préférence entre pH 6,4 et 6,9.

29. Procédé pour la production d'éthanol par fermentation bactérienne de la bactérie Gram-positive suivant l'une quelconque des revendications 9 à 19, comprenant l'utilisation de conditions optimisées de fermentation dans lesquelles la température du milieu de fermentation est comprise dans l'intervalle de 40 à 75°C.

30. Procédé suivant la revendication 29, dans lequel la température du milieu de fermentation est comprise de préférence entre 52 et 70°C.

31. Procédé suivant la revendication 29 ou 30, dans lequel la température du milieu de fermentation est comprise de préférence entre 60 et 68°C.

32. Procédé pour la production continue d'éthanol par fermentation bactérienne de la bactérie Gram-positive suivant l'une quelconque des revendications 9 à 19, dans lequel les vitesses de dilution de la charge d'alimentation sont comprises entre 0,3 et 0,8 h⁻¹.

33. Procédé suivant la revendication 32, dans lequel les vitesses de dilution de la charge d'alimentation sont comprises de préférence entre 0,4 et 0,6 h⁻¹.

34. Procédé suivant l'une quelconque des revendications 26 à 33, dans lequel la bactérie utilisée est la souche de *Bacillus* TN.
